(19) Europäisches Patentamt
European Patent Office
Office européen des brevets

(11) EP 0 648 106 B1

(12) **FASCICULE DE BREVET EUROPEEN**

(45) Date de publication et mention de la délivrance du brevet:
**15.12.1999 Bulletin 1999/50**

(21) Numéro de dépôt: **94915197.1**

(22) Date de dépôt: **02.05.1994**

(51) Int. Cl.$^6$: **A61K 7/06**, A61K 7/48

(86) Numéro de dépôt international:
**PCT/FR94/00499**

(87) Numéro de publication internationale:
**WO 94/26238 (24.11.1994 Gazette 1994/26)**

(54) **UTILISATION D'UN TERPOLYMERE DERIVE DE VINYL LACTAME COMME AGENT DE MOUSSAGE DANS DES COMPOSITIONS FORMANT UNE MOUSSE AEROSOL ET COMPOSITION DE MISE EN UVRE**

VERWENDUNG EINES VON VINYLLACTAM ABGELEITETEN TERPOLYMERS ALS SCHAUMBILDNER IN ZUSAMMENSETZUNGEN, DIE EINEN AEROSOLSCHAUM BILDEN, UND VERWENDETE ZUSAMMENSETZUNG

USE OF A VINYL LACTAM DERIVED TERPOLYMER AS FOAMING AGENT IN AEROSOL FOAM-FORMING COMPOSITIONS, AND COMPOSITION THEREBY OBTAINED

(84) Etats contractants désignés:
**DE ES FR GB IT**

(30) Priorité: **07.05.1993 FR 9305523**

(43) Date de publication de la demande:
**19.04.1995 Bulletin 1995/16**

(73) Titulaire: **L'OREAL**
**75008 Paris (FR)**

(72) Inventeur: **DUPUIS, Christine**
**F-75018 Paris (FR)**

(74) Mandataire: **Casalonga, Axel**
**BUREAU D.A. CASALONGA - JOSSE**
**8, avenue Percier**
**75008 Paris (FR)**

(56) Documents cités:

| | |
|---|---|
| **EP-A- 0 523 388** | **DE-A- 2 016 595** |
| **DE-A- 2 109 522** | **FR-A- 2 679 444** |
| **US-A- 3 405 084** | **US-A- 4 482 534** |
| **US-A- 5 015 708** | |

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Printed by Xerox (UK) Business Services
2.16.7/3.6

EP 0 648 106 B1

## Description

La présente invention est relative à l'utilisation d'un terpolymère dérivé de vinyl lactame dans des compositions destinées au traitement des cheveux et/ou de la peau, pressurisées en aérosol et formant une mousse à la sortie du dispositif aérosol.

Des compositions cosmétiques pressurisées dans des dispositifs aérosol, dans des conditions telles à former à la sortie une mousse, sont bien connues et sont utilisées notamment dans le traitement des cheveux et/ou de la peau.

On appellera de telles compositions dans la suite de la description "mousse aérosol".

Ces mousses permettent généralement d'obtenir sur les cheveux une bonne répartition des compositions cosmétiques et elles sont en outre d'utilisation aisée, plus économique au niveau des produits.

Ces mousses doivent être suffisamment stables pour ne pas se liquéfier rapidement et doivent également disparaître rapidement lors du massage servant à faire pénétrer la composition dans la chevelure ou à répartir celle-ci sur la peau.

Pour former des mousses, on peut utiliser des agents tensio-actifs anioniques, non-ioniques ou amphotères, mais, utilisés seuls, ces agents de surface produisent des mousses de qualité non satisfaisante, dans la mesure où, soient elles se liquéfient au moment de l'application, soit elles ne disparaissent pas après l'application au moment du massage.

Un autre moyen pour former des mousses est d'utiliser des polymères moussants, mais, dans ce cas, on se heurte à des difficultés de solubilisation des polymères dans l'eau ou, si ces polymères sont suffisamment solubles, on obtient alors des mousses dont les propriétés cosmétiques peuvent encore être améliorées.

La demanderesse a découvert que l'utilisation d'un terpolymère constitué de :

- 25 à 90% de vinyl lactame,
- 1 à 55% d'acide carboxylique insaturé
- 1 à 20% de (méth)acrylate d'alkyle, comportant au moins 6 atomes de carbone,

permettait d'obtenir une mousse conférant du plixant à la chevelure (tenue de la coiffure) et de la douceur aux cheveux. La mousse ainsi obtenue confère également à la peau une douceur améliorée.

Le document US 3,405,084 décrit des terpolymères comportant 25 à 75% de vinylpyrrolidone, 20 à 70% d'acrylate et 3 à 25% d'un acide insaturé, terpolymère utilisé dans des sprays comme agents fixateurs. Le document EP 0,523,382 a pour objet une composition aérosole de préférence sous forme de spray contenant un terpolymère de vinylpyrrolidone, de méthacrylate d'éthyle et d'acide méthacrylique. Le document US A 4,482,534 décrit des compositions contenant un terpolymère de vinylpyrrolidone, de méthacrylate de lauryl et d'acide acrylique, et en outre 3% de nitroglycérine.

Les mousses obtenues conformément à l'invention sont particulièrement stables et rigides pour leur utilisation dans le traitement cosmétique de la peau et des cheveux, elles possèdent des propriétés filmogènes et sont particulièrement intéressantes par la douceur qu'elles confèrent aux cheveux et à la peau.

Ces mousses constituent également un véhicule particulièrement approprié pour appliquer sur la peau ou les cheveux des agents actifs cosmétiques ou dermatologiques.

On appellera dans la suite de la description un "traitement cosmétique", tout traitement qui vise à améliorer l'esthétique ou l'état de surface de la peau ou des cheveux.

On appellera "traitement dermatologique", un traitement mettant en oeuvre des substances actives ayant un effet thérapeutique ou préventif d'une maladie de la peau.

L'invention a donc pour objet l'utilisation à titre d'agent de moussage, d'un copolymère dérivé de vinyl lactame défini ci-après.

Un autre objet de l'invention est constitué par les compositions cosmétiques destinées au traitement de la peau ou des cheveux, appliquées sour forme d'une mousse à partir d'un dispositif aérosol pour le traitement de la peau ou des cheveux.

L'invention a également pour objet un procédé de traitement de la peau et des cheveux mettant en oeuvre une telle mousse.

D'autres objets de l'invention apparaîtront à la lecture de la description et des exemples qui suivent.

Le copolymère utilisé comme agent de moussage dans des compositions destinées à être appliquées sur la peau ou les cheveux, pour former une mousse à partir d'une composition conditionnée dans un dispositif aérosol, est un terpolymère constitué de :

- 25 à 90% de vinyl lactame,
- 1 à 55% d'acide carboxylique, insaturé
- 1 à 20% d'un (méth)acrylate d'alkyle comportant au moins 6 atomes de carbone.

Le vinyl lactame est en particulier la 2-vinylpyrrolidone.

L'acide carboxylique insaturé est choisi de préférence parmi les acides acrylique, méthacrylique, itaconique ou crotonique.

L'acrylate ou le méthacrylate d'alkyle comportant au moins 6 atomes de carbone, est de préférence un ester acrylique ou méthacrylique comportant de 8 à 18 atomes de carbone.

Les groupements alkyle sont choisis de préférence parmi les groupements 2-éthyl hexyle, octyle, lauryle et stéaryle.

Les polymères utilisables conformément à l'invention, sont connus en eux-mêmes et peuvent être préparés par exemple selon le procédé décrit dans le brevet US 5.015.708.

Les terpolymères utilisables conformément à l'invention, sont de préférence choisis parmi les polymères comportant :

- 40 à 70% de vinylpyrrolidone,
- 15 à 40% d'acide carboxylique insaturé, et
- 5 à 20% de (méth)acrylate d'alkyle, ayant un nombre d'atomes de carbone compris entre 8 et 18.

Les copolymères particulièrement préférés sont choisis parmi les terpolymères de vinylpyrrolidone, d'acide (méth)acrylique et de préférence d'acide acrylique et de méthacrylate de lauryle.

Un polymère particulièrement préféré est le polymère commercialisé sous la dénomination "ANIONIC VP TERPOLYMER" par la Société ISP, comprenant 66 à 68% en poids de vinylpyrrolidone, 23 à 25% d'acide acrylique et 9% de méthacrylate de lauryle.

Le terpolymère utilisé conformément à l'invention est utilisé comme agent de moussage unique ou complémentaire.

On l'appelle agent de moussage unique lorsque le polymère est utilisé seul pour former la mousse; on considère qu'il intervient comme agent de moussage complémentaire, lorsqu'il est utilisé conjointement avec d'autres composés susceptibles de mousser.

La mousse obtenue après expansion à l'air libre à partir du dispositif aérosol, grâce à l'utilisation du terpolymère défini ci-dessus, a une masse volumique inférieure ou égale à 0,3 g/cm$^3$ à 20°C.

La masse volumique est déterminée de la façon suivante :

On conditionne une solution aqueuse à 1% de terpolymère dans un boîtier aérosol constitué par un boîtier aluminium monobloc de (45 x 28) avec une valve, précision P73, sans tube plongeur, ayant un poussoir diffuseur axial pour coupelle conique, 021550. Le boîtier aérosol est rempli à raison de 90 g de solution à 1% de terpolymère et 10 g de gaz propulseur AEROGAZ 3,2N constitué de butane, isobutane > 55% et propane, vendu par la Société ELF AQUITAINE. La manipulation est effectuée 24 heures après la pressurisation de l'aérosol en salle conditionnée à 20°C ± 1°C. Le matériel et l'échantillon sont à la même température. Un godet cylindrique est pesé à vide (soit P1 son poids) puis directement rempli de la mousse produite par l'aérosol. Chaque boîtier aérosol est bien agité avant l'emploi de façon à émulsionner le gaz propulseur.

Pour une répartition uniforme de la mousse dans le godet, les aérosols sont utilisés tête en bas dans un mouvement tournant et régulier.

Dès la fin de l'expansion de la mousse, on l'arase immédiatement et rapidement à l'aide d'une spatule large et on pèse à nouveau le godet, soit P2 son poids.

On détermine la masse volumique de la mousse selon la formule suivante :

$$\text{masse volumique à } 20°C = P2 - P1/V \text{ (V est le volume du godet).}$$

On effectue trois déterminations pour chaque polymère. La valeur retenue est la valeur moyenne des ces déterminations en g/cm$^3$.

La mousse obtenue grâce à l'utilisation du terpolymère conforme à l'invention présente, par ailleurs, une stabilité particulièrement améliorée, supérieure à 5 minutes.

La stabilité de mousse est mesurée en introduisant dans une éprouvette graduée de 50 ml, 5 g de mousse obtenue à partir d'une solution aqueuse à 1% de polymère, conditionnée comme décrit ci-dessus, puis l'on mesure au bout de combien de temps la mousse s'est liquéfiée en donnant 5 mi de liquide.

La mousse obtenue conformément à l'invention, est particulièrement rigide, c'est-à-dire qu'elle ne se liquéfie pas au moment de l'application sur les cheveux ou la peau, mais disparaît rapidement lors du massage. Ce temps de disparition est généralement compris ente 10 et 40 secondes.

Le terpolymère à base de vinyl lactame, tel que défini ci-dessus, est généralement utilisé dans un milieu approprié pour une application sur les cheveux ou la peau et cosmétiquement acceptable. Le terpolymère est présent dans ce milieu dans des proportions comprises entre 0,05 et 10% en poids par rapport au poids total de la composition, et

de préférence entre 0,2 et 3%.

La composition introduite dans le dispositif aérosol et qui, après expansion, forme la mousse aérosol, constitue un autre objet de l'invention. Elle est caractérisée par le fait qu'elle contient, dans les proportions précitées, le terpolymère à base de vinyl lactame tel que défini ci-dessus, dans un milieu aqueux cosmétiquement acceptable et dans des proportions suffisantes pour former une mousse rigide et stable après expansion à l'air à partir du dispositif aérosol.

Les milieux cosmétiques aqueux peuvent contenir en plus de l'eau, tout solvant cosmétiquement acceptable choisi en particulier parmi les monoalcools tels que les alcanols ayant de 1 à 8 atomes de carbone, comme l'éthanol, l'isopropanol, l'alcool benzylique, l'alcool phényléthylique; les polyalcools tels que les alkylèneglycols comme l'éthylèneglycol; les éthers de glycol comme les mono- di- et triéthylèneglycol; les alkyléthers comme par exemple l'éthylèneglycol monométhyléther, l'éthylèneglycol monoéthyléther, le diéthylèneglycol monoéthyléther, utilisés seuls ou en mélange.

Ces solvants, lorsqu'ils sont présents, sont utilisés dans des proportions inférieures ou égales à 50% en poids, et de préférence inférieures à 30% en poids par rapport au poids total de la composition, et dans des quantités telles à permettre l'obtention avec le terpolymère dérivé de vinyl lactame défini ci-dessus, une mousse ayant une masse volumique inférieure ou égale à 0,3 g/cm$^3$ à 20°C et une stabilité supérieure à 5 minutes, dans ce milieu cosmétiquement acceptable.

Les compositions conformes à l'invention peuvent contenir des agents destinés au traitement cosmétique des cheveux et/ou de la peau, ou des substances dermatologiquement actives.

Ces agents destinés au traitement cosmétique des cheveux et/ou de la peau, peuvent avoir des propriétés moussantes ou non lorsqu'ils sont utilisés isolément dans le milieu cosmétique ne contenant pas le terpolymère dérivé de vinyl lactame tel que défini ci-dessus.

On considère qu'un agent cosmétique ne mousse pas lorsqu'il ne forme pas de mousse ou lorsque la masse volumique de la mousse aérosol est supérieure à 0,3 g/cm$^3$ à 20°C, selon le test décrit ci-dessus pour le terpolymère dérivé de vinyl lactame.

Les agents cosmétiquement actifs peuvent être des produits ayant pour but d'améliorer la brillance, le toucher, le démêlage, la tenue, de donner un effet anti-gras, antipelliculaire ou encore pour renforcer, déformer ou conditionner les cheveux.

Parmi ces agents cosmétiquement actifs, on peut citer les agents cationiques traitants, constitués par des agents tensio-actifs ou des polymères.

Parmi ces composés, on peut citer plus particulièrement :

- les dérivés d'amines grasses comme les alkyl($C_{12}$-$C_{20}$)amidopropyldiméthylamines;
- les sels comme les acétates d'alkylamines, des sels d'ammonium quaternaires tels que des chlorures, bromures ou hydrogénophosphates d'alkyldiméthylhydroxyéthylammonium, le chlorure d'acétyldiméthyldodécylammonium, des méthosulfates d'alkylamidoéthyltriméthylammonium, les lactates de N,N-diméthylamino ou N,N-diéthylaminopolyoxyéthylcarboxylate oxyéthyléné par exemple avec 4 moles d'oxyde d'éthylène, des sels d'alcoylpyridinium tels que le chlorure de 1-(2-hydroxyéthyl)carbamoylméthylpyridinium, le chlorure de N-lauryl, colaminoformylméthylpyridinium, les dérivés d'imidazoline tels que les alcoylimidazolines;
- les oxydes d'amines, les oxydes d'alkyldiméthylamines, les oxydes d'alkylaminoéthyldiméthylamines et les oxydes d'alkylamidopropyldiméthylamines.

Les dérivés cationiques répondant à la formule :

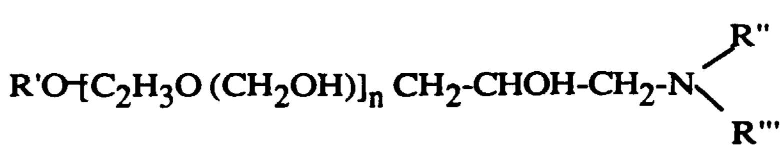

(I)

dans laquelle :

R' désigne un radical alcoyle linéaire ou ramifié, saturé ou insaturé, un radical alkylaryle à chaîne alkyle linéaire ou ramifiée, comportant de 8 à 22 atomes de carbone;
R'' et R''' désignent des radicaux hydroxyalkyle inférieurs ou des radicaux alkylènes réunis entre eux pour former un hétérocycle;
n est un nombre compris entre 0,5 et 10.

D'autres agents de surface cationiques utilisables sont les composés dispersibles dans l'eau de formule (II) :

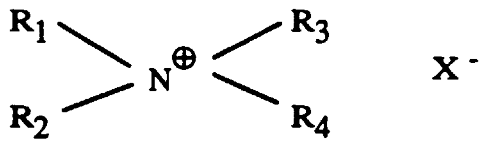

(II)

dans laquelle :

a) Lorsque $R_1$ désigne le groupement de formule :

$$R_5\text{-O-}[C_2H_3(R_6)O]_p[C_2H_3(CH_2OH)O]_{n-1}CH_2\text{-CHOH-}CH_2\text{-}$$

dans laquelle :

$R_5$ désigne un radical aliphatique linéaire ou ramifié, saturé ou insaturé;
$R_6$ est un radical alkyle, un radical alcoxyméthyle linéaire ou ramifié, un radical alcényloxy linéaire;
p désigne un nombre entier ou décimal de 1 à 2,5;
n désigne un nombre entier ou décimal de 2 à 20;
$R_2$ désigne un radical alkyle ou hydroxyalkyle ayant de 1 à 3 atomes de carbone;
$R_3$ et $R_4$, identiques ou différents, désignent un radical alcoyle ou hydroxyalcoyle ayant de 1 à 3 atomes de carbone, ou bien forment avec l'atome d'azote auquel ils sont rattachés, un hétérocycle à 5 ou 6 chaînons;
$X^-$ désigne un anion et de préférence un anion méthylsulfate, méthanesulfonate, p-toluènesulfonate, bromure, chlorure ou iodure.

b) Lorsque $R_2$ et $R_3$ désignent un radical méthyle;

$R_1$ et $R_4$ ont les significations suivantes :

(i) $R_1$ et $R_4$ désignent un radical aliphatique linéaire;
(ii) ou bien $R_1$ désigne un radical aliphatique linéaire saturé et $R_4$ désigne un radical méthyle, hydroxyéthyle, benzyle, ou un reste de panthénol;
(iii) ou bien $R_1$ désigne un radical alkylamidopropyle (alkyle en $C_{14}$-$C_{22}$) et $R_4$ désigne un groupe alkylacétate (alkyle en $C_{12}$-$C_{16}$).

$X^-$ désigne un anion tel qu'un halogénure ou $CH_3SO_4^-$ .

c) Lorsque $R_1$ désigne un groupe alkylamidoéthyle et/ou alcénylamidoéthyle, dans lesquels le radical alkyle et/ou alcényle contenant de 14 à 22 atomes de carbone dérive des acides gras de suif et $R_2$ et $R_3$ forment avec l'azote un hétérocycle substitué du type 4,5-dihydroimidazole;

$R_4$ désigne un alkyle en $C_1$-$C_4$;
$X^-$ désigne un anion $CH_3SO4^-$ .

Les dérivés d'ammonium bis-quaternaire à deux chaînes lipophiles, choisis parmi :

a) ceux de formule :

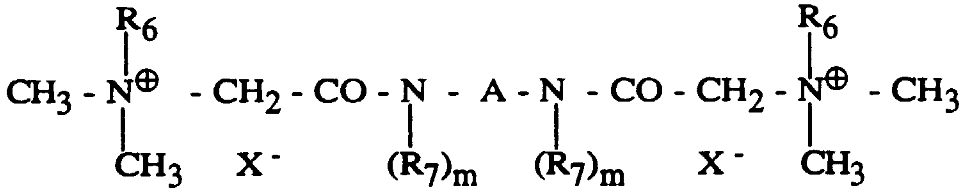

(III)

dans laquelle :

$R_6$ désigne un groupement aliphatique saturé ou insaturé, linéaire ou ramifié, ayant de 8 à 22 atomes de carbone ou le mélange de ces groupements ou un mélange de chaînes lipophiles dérivant de produits naturels, ayant de 8 à 30 atomes de carbone;
A désigne un groupement $-(CH_2)-_n$ , dans lequel n désigne un nombre entier de 1 à 18;
$R_7$ désigne H; et
m = 1;
A peut également former avec les atomes d'azote auxquels il est relié, un groupement hétérocyclique, auquel cas m = O;
$X^-$ désigne un anion dérivant d'un acide minéral ou organique.

Ces composés sont décrits dans le brevet français de la demanderesse n° 2.464.710.

b) Les $\alpha,\omega$-bis(alkyle en $C_{16}$-$C_{18}$ diméthylammonio)hydroxyalkylène.

Parmi les agents tensio-actifs cationiques plus particulièrement préférés, on peut citer :

(1) les composés de formule :

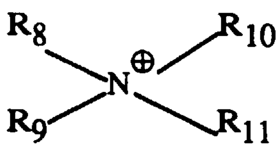 $X^-$ (IV)

dans laquelle :

$R_8$ et $R_{11}$ désignent chacun des radicaux alkyle en $C_{16}$-$C_{18}$ ou un mélange de radicaux alcényle et/ou alkyle dérivés des acides gras du suif, ayant 14 à 22 atomes de carbone; et
$R_9$ et $R_{10}$ désignent le radical méthyle;
$X^-$ désigne l'ion $Cl^-$ ;
ou bien $R_8$ désigne un radical alkyle en $C_{18}$ ou un reste de panthénol, $R_{11}$ désigne un radical benzyle, $R_9$ et $R_{10}$ désignent le radical méthyle, et $X^-$ désigne $Cl^-$ .

(2) les dérivés de bis-ammonium quaternaire portant un groupement ester, tels que le produit vendu sous la dénomination "AMONYL DM" par la Société SEPPIC.
(3) le dérivé d'ammonium bis-quaternaire répondant à la formule :

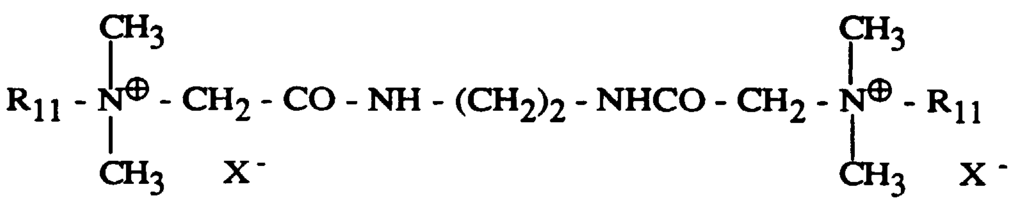 (V)

dans laquelle $R_{11}$ est une chaîne suif et $X^-$ est $Cl^-$ .
(4) le chlorure d'alkyle (suif) triméthylammonium, vendu en solution dans l'alcool isopropylique sous la dénomination "ARQUAT T 50" par la Société ARMAK, les chlorure ou bromure de cétyl triméthylammonium, le bromure de myristyl triméthylammonium.
(5) le 2-hydroxy 1,3-bis(stéaryldiméthylammonium)propane, vendu en solution hydroalcoolique sous la dénomination "M. QUAT. DIMER 18" par la Société MAZER CHEMICALS.
(6) le méthosulfate de 1-méthyl 2-alkyl 3-alkylamidoéthylimidazolinium, dans lequel le groupe alkyle dérive des acides gras de suif, vendu sous la dénomination "REWOQUAT W 7500" par la Société REWO.
(7) le chlorure de cocoylamidopropyl diméthylacétamido ammonium, répondant à la formule :

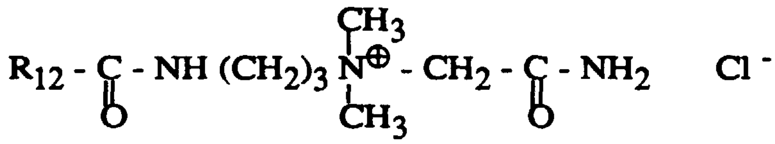

(VI)

où le radical $R_{12}$ est une chaîne coprah.

(8) l'éthylsulfate d'acylamidopropyl diméthyléthylammonium, dans lequel le groupement acyle dérive des acides gras de lanoline, vendu sous la dénomination "LANOQUAT 50" et référencé sous le nom de QUATERNIUM 33 dans le dictionnaire CTFA.

(9) le chlorure de $\gamma$-gluconamidopropyldiméthyl hydroxyéthylammonium, vendu sous la dénomination de "CERAPHYL 60" par la Société VAN DYK et référencé sous le nom de QUATERNIUM 22 dans le dictionnaire CTFA.

(10) le chlorure de triméthyldocosyl ammonium.

(11) le phosphate de cétyldiméthyl (2'-hydroxyéthyl)ammonium, vendu sous la dénomination "LUVIQUAT MONO cp" par la Société BASF.

(12) l'oxyde de stéaryldiméthylamine et l'oxyde d'alkyl(coprah) amidopropyldiméthylamine.

(13) la stéarylamidopropyldiméthylamine.

On peut également utiliser comme agent cosmétique actif, des polymères cationiques qui sont choisis parmi les polymères comportant des groupements amine primaire, secondaire, tertiaire et/ou quaternaire, faisant partie de la chaîne polymère ou directement reliés à celle-ci, par l'intermédiaire d'un groupement hydrocarboné ayant un poids moléculaire compris entre 500 et environ 5.000.000, et de préférence entre 1.000 et 3.000.000.

Parmi ces polymères, on peut citer plus particulièrement les protéines quaternisées, les polysiloxanes quaternisés et les polymères du type polyamine, polyaminoamide, polyammonium quaternaire.

**A.** Les protéines quaternisées sont en particulier des polypeptides modifiés chimiquement et portant en bout de chaîne ou greffés sur celle-ci, des groupements ammonium quaternaires.

**B.** Une autre famille de polymères cationiques est constituée par les polymères cationiques siliconés. Ces polymères cationiques siliconés sont des polydiméthylsiloxanes ou des polydiphénylsiloxanes ou encore des polyméthylphénylsiloxanes comportant au moins en bout de chaîne ou pendante une chaîne hydrocarbonée interrompue par un ou plusieurs groupes amino.

**C.** Les polymères du type polyamine, polyaminoamide ou polyammonium quaternaire, utilisables conformément à la présente invention, sont décrits en particulier dans les brevets français de la demanderesse n° 82 07 996 ou 84 04 475.

Parmi ces polymères, on peut citer :

(1) Les copolymères vinylpyrrolidone-acrylate ou méthacrylate de dialkylaminoalkyle, quaternisés ou non, tels que les produits vendus sous la dénomination "GAFQUAT" par la Société GAF CORPORATION, comme par exemple "GAFQUAT 734 ou 735", ou bien les produits dénommés "COPOLYMER 845, 958 et 937". Ces polymères sont décrits en détail dans le brevet français 2.077.143 et 2.393.573. On cite aussi le GAFFIX VC-713, vendu par la Société GAF, qui est un copolymère de vinylcaprolactame/vinylpyrrolidone/méthacrylate de diméthylaminoéthyle.

(2) Les dérivés d'éthers de cellulose comportant des groupements ammonium quaternaires, décrits dans le brevet français 1.492.597, et en particulier les polymères commercialisés sous les dénominations "JR" (JR 400, JR 125, JR 30M) ou "LR" (LR 400, LR 30M) par la Société UNION CARBIDE CORPORATION. Les polymères sont également définis dans le dictionnaire CTFA comme des ammonium quaternaires d'hydroxyéthylcellulose ayant réagi avec un époxyde substitué par un groupement triméthylammonium.

(3) Les dérivés de cellulose cationiques tels que les copolymères de cellulose ou les dérivés de cellulose greffés par un monomère hydrosoluble d'ammonium quaternaire et décrits plus en détail dans le brevet américain 4.131.576, tels que les hydroxyalkylcelluloses comme les hydroxyméthyl-, hydroxyéthyl- ou hydroxypropyl celluloses greffées par un sel de méthacryloyloxyéthyl triméthylammonium, méthacrylamidopropyl triméthylammonium, diméthyldiallylammonium.

Les produits commercialisés répondant à cette définition sont plus particulièrement les produits vendus sous les dénominations "CELQUAT L 200" et "CELQUAT H 100" par la Société NATIONAL STARCH.

(4) Les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles

aromatiques ou hétérocycliques, ainsi que les produits d'oxydation et/ou de quaternisation de ces polymères. De tels polymères sont décrits dans les brevets français 2.162.025 et 2.280.361.

(5) Les polyaminoamides solubles dans l'eau, préparés en particulier par polycondensation d'un composé acide avec une polyamine. Ces polyaminoamides peuvent éventuellement être réticulés et/ou alcoylés. De tels polymères sont décrits en particulier dans les brevets français 2.252.840, 2.368.508 et 1.583.363, ainsi que dans les brevets américains 3.227.615 et 2.961.347.

D'autres polymères cationiques utilisables conformément à l'invention, sont les dérivés de la chitine comme le dérivé quaternaire LEXQUAT.CH, vendu par la Société INOLEX, qui est le produit de réaction du chitosane et de l'oxyde de propylène, quaternisé à l'épichlorhydrine et le dérivé cationique KYTAMER PC, vendu par la Société AMERCHOL, qui est le pyrrolidone carboxylate de chitosane.

Les agents traitants peuvent également être de nature anionique et plus particulièrement des polymères anioniques utilisés seuls ou en association avec les agents traitants cationiques définis ci-dessus.

Une forme de réalisation particulièrement préférée consiste à utiliser en association des polymères cationiques et des polymères anioniques comme décrit dans les brevets français 2.542.997, 2.544.000, 2.521.427, 2.383.660.

Les polymères anioniques comportent des unités acides carboxylique, sulfonique ou phosphorique et ont un poids moléculaire compris entre 500 et 5.000.000, et de préférence entre 1.000 et 3.000.000.

Parmi ces polymères, on peut plus particulièrement citer :

- les sels alcalins d'acides polyhydroxycarboxyliques, tels que les produits vendus sous la dénomination "HYDAGEN F" par la Société HENKEL;
- les homopolymères d'acides acrylique ou méthacrylique de préférence non réticulés ou leurs sels, tels que par exemple les produits vendus sous les dénominations "VERSICOL E ou K" par la Société ALLIED COLLOID, ou le produit vendu sous la dénomination "DARVAN N°7" par la Société VAN DER BILT;
- les copolymères acide méthacrylique/méthacrylate d'alkyle en $C_1$-$C_4$;
- les copolymères dérivés d'acides ou d'anhydrides maléique, fumarique, itaconique et d'esters vinyliques, d'éthers vinyliques, d'halogénures vinyliques, de dérivés phénylvinyliques, d'acides acryliques ou d'acrylates, ces copolymères étant éventuellement partiellement ou totalement estérifiés et décrits plus particulièrement dans les brevets US 2.047.398, 2.723.248, 2.102.213, le brevet britannique 839.805. Parmi ces copolymères, on peut plus particulièrement citer les produits vendus sous les dénominations "GANTREZ" "AN ou ES" par la Société GENERAL ANILINE ou "EMA 1325" par la Société MONSANTO. D'autres polymères appartenant à cette classe sont des copolymères d'anhydrides maléique, fumarique, itaconique et d'un ester allylique ou méthallylique, et éventuellement d'acrylamide, de méthacrylamide, d'une alpha oléfine, des acides acrylique ou méthacrylique ou leurs esters ou de vinylpyrrolidone; les fonctions anhydrides étant mono-estérifiées ou mono-amidifiées comme décrit dans les demandes de brevets français publiées 76 13 929, 76 20 917;
- les terpolymères constitués de 10 à 91% en poids d'acétate de vinyle, de 3 à 20% en poids d'un acide carboxylique insaturé choisi de préférence parmi l'acide crotonique, l'acide allyloxy acétique, l'acide allyloxy propionique et l'acide vinylacétique et de 4 à 60% en poids d'au moins un ester vinylique, allylique ou méthallylique d'un acide carboxylique alpha-cyclique. Parmi ces polymères, on peut citer de préférence le copolymère acétate de vinyle/acide crotonique/t-butylbenzoate de vinyle (65/10/25) tel que décrit dans le brevet français n° 2.439.798;
- les copolymères acétate de vinyle/acide crotonique greffés sur le polyéthylèneglycol, tel que le produit vendu par la Société HOECHST sous la dénomination "ARISTOFLEX A";
- les terpolymères acétate de vinyle/acide crotonique/vinylnéodécanoate, tels que le produit vendu sous la dénomination "RESYN 28-29-30" par la Société NATIONAL STARCH;
- les polyacrylamides comportant des groupements carboxylates, tels que le produit vendu sous la dénomination "CYANAMER A 370" par la Société AMERICAIN CYANAMID.

Les polymères comportant des motifs sulfoniques sont plus particulièrement choisis parmi les sels de sodium de l'acide polystyrène sulfonique, tels que les produits vendus sous les dénominations "FLEXAN 500" et "FLEXAN 130" ayant respectivement un poids moléculaire d'environ 500.000 et 800.000, par la Société NATIONAL STARCH.

- les sels de polyacrylamide sulfoniques, tels que l'acide polyacrylamido méthylpropane sulfonique comme le produit vendu sous la dénomination "COSMEDIA POLYMER HSP 1180" par la Société HENKEL;
- les sels d'un polymère contenant des motifs acides alkylnaphtalène sulfoniques, comme le produit vendu sous la dénomination "DARVAN N°1" par la Société VAN DER BILT;
- les polyvinylsulfonates de sodium ayant un poids moléculaire compris entre 1.000 et 100.000.

D'autres polymères anioniques tels que le copolymère réticulé d'acrylamide/acrylate d'ammonium (5/95 en poids) en émulsion eau-dans-huile, vendu par la Société HOECHST sous la dénomination "BOZEPOL C" ou le copolymère réticulé d'acrylamide/acide 2-acrylamido 2-méthylpropane sulfonique, partiellement ou totalement neutralisé, en émulsion huile-dans-eau.

Des agents traitants non ioniques peuvent être constitués par des polymères non ioniques ayant un poids moléculaire compris entre 500 et 3.000.000, pouvant être utilisés seuls ou en mélange avec des polymères anioniques et/ou cationiques mentionnés ci-dessus.

Parmi ces polymères non ioniques, on peut citer :

- la polyvinylpyrrolidone,
- les copolymères de vinylpyrrolidone/acétate de vinyle.
- les polyacrylamides,
- les polyéthylèneglycols,
- les celluloses non-ioniques modifiées, hydrophobes, comme le produit vendu sous la dénomination "NATROSOL Grade Plus 330 cs" par la Société AQUALON, ou sous la dénomination "AMERCEL HM 1500" par la Société AMERCHOL,
- les polyamides hydrosolubles, décrits plus particulièrement dans le brevet US-A-4.082.730 et dans le brevet français 2.508.795 et de préférence les poly-β-alanines.

Les polymères amphotères peuvent être utilisés également comme agents traitants seuls ou en association avec les dérivés cationiques ou les polymères mentionnés ci-dessus. Ces polymères ont un poids moléculaire de 500 à 3.000.000 et sont choisis en particulier parmi les polymères comportant des motifs A et B répartis statistiquement dans la chaîne polymère où A désigne un motif dérivant d'un monomère comportant au moins un atome d'azote basique et B désigne un motif dérivant d'un monomère acide comportant un ou plusieurs groupements carboxyliques ou sulfoniques, A et/ou B peuvent désigner également des groupements dérivant de monomères zwittérioniques de carboxybétaïne ou de sulfobétaïne.

A et B peuvent également désigner des chaînes polymères cationiques comportant des groupements amine primaire, secondaire, tertiaire ou quaternaire, dans lesquelles l'un des groupements amine porte un groupement carboxylique ou sulfonique relié par l'intermédiaire d'un radical hydrocarboné; ou bien A et B font partie d'une chaîne d'un polymère à motif éthylène α, β-dicarboxylique dont l'un des groupements carboxylique a été amené à réagir avec une polyamine comportant un ou plusieurs groupements amine primaire ou secondaire.

Des polymères amphotères particulièrement préférés sont choisis parmi :

- les polymères résultant de la réaction d'un polyaminoamide obtenu par polycondensation d'acide adipique et de diéthylènetriamine en quantité équimoléculaire et réticulé avec l'épichlorhydrine à raison de 11 moles de réticulant pour 100 groupements amine secondaire du polyaminoamide, alcoylé avec la propanesultone dans des proportions de 50% ou bien avec le chloracétate de soude;
- le polymère obtenu par polycondensation de l'épichlorhydrine et de la pipérazine en présence de soude et bétaïnisé; - les copolymères d'acide acrylique et de chlorure de diméthyldiallylammonium, vendus sous les dénominations "MERQUAT 280" et "MERQUAT 295";
- Le copolymère d'octylacrylamide/acrylate/butylaminoéthylméthacrylate, vendu sous la dénomination "AMPHOMER" par la Société NATIONAL STARCH;
- le copolymère méthacrylate de méthyle/méthacrylate de carboxyméthyldiméthylammonioéthyle;
- les copolymères de méthacrylate d'alkyle($C_1$-$C_{18}$)/méthacrylate de carboxyméthyldiméthylammoniométhyle.

A titre d'exemple, on peut citer le produit vendu sous la dénomination "AMPHOSET" par la Société MITSUBISHI PETROCHEMICAL Co., Ltd, ou sous le nom de "AMERSETTE" par la Société AMERCHOL, ayant un poids moléculaire de 70.000 à 90.000.

Les polymères dérivés du chitosane décrits en particulier dans le brevet français 2.137.684, résultent de l'acylation partielle du chitosane par un diacide carboxylique.

A titre d'exemple, on peut citer le polymère comportant 0 à 20% de motif acétylchitosane, 40 à 50% de motif chitosane et 40 à 50% de motif chitosane acylé par l'acide succinique.

Les compositions conformes à l'invention peuvent également contenir des silicones volatiles ou non volatiles, non cationiques.

Les silicones volatiles possèdent un point d'ébullition compris entre 60 et 260°C. Les silicones non volatiles sont choisies plus particulièrement parmi les polyalkylsiloxanes, les polyarylsiloxanes, les polyalkylarylsiloxanes, les copolymères de polyéthersiloxane organomodifiés ou non, les gommes et résines de silicones, des polysiloxanes organomodifiés, ainsi que leurs mélanges.

Les silicones volatiles sont choisies plus particulièrement parmi des silicones cycliques comportant de 3 à 7 atomes de silicium, telles que l'octaméthylcyclotétrasiloxane ou le décaméthylcyclopentasiloxane, et les cyclopolymères tels que le diméthylsiloxane/méthylalkylsiloxane, ou encore des silicones volatiles linéaires ayant 3 à 9 atomes de silicium, telles que l'hexaméthyldisiloxane.

Les silicones non volatiles sont choisies parmi :

a/ les polyalkylsiloxanes, telles que les polydiméthylsiloxanes linéaires à groupements terminaux triméthylsilyle, ayant une viscosité de $5 \times 10^{-6}$ à 2,5 $m^2/s$. à 25°C, et les polydiméthylsiloxanes linéaires à groupements terminaux trihydroxysilyle et les polyalkyl($C_1$-$C_{20}$) siloxanes;
b/ les polyalkylarylsiloxanes choisis parmi les polyméthylphénylsiloxanes ou les polydiméthyldiphénylsiloxanes linéaires et/ou ramifiés, ayant une viscosité de $10^{-5}$ à $5 \times 10^{-2}$ $m^2/s$. à 25°C;
c/ les copolymères de polyéthersiloxane modifiés ou non;
d/ les gommes de silicone constituées de polydiorganosiloxane à poids moléculaire élevé compris entre 200.000 et 1.000.000, utilisées seules ou en mélange avec un solvant choisi parmi les silicones volatiles, les huiles polydiméthylsiloxane (PDMS), les huiles polyphénylméthylsiloxane (PPMS), les isoparaffines, le chlorure de méthylène, le pentane, le dodécane, le tridécane, le tétradécane ou leurs mélanges;
e/ les résines d'organopolysiloxane qui ont des systèmes siloxaniques réticulés renfermant des unités $R_2SiO_2/_2$, $RSiO_{3/2}$ et $SiO_{4/2}$ , dans lesquelles R représente un groupement hydrocarboné possédant de 1 à 6 atomes de carbone ou un groupement phényle;
f/ les silicones organomodifiées définies ci-dessus et comportant sur leur structure générale un ou plusieurs groupements organofonctionnels directement fixés sur la chaîne siloxanique ou fixés par l'intermédiaire d'un radical hydrocarboné, choisi parmi :

(i) des groupements thiols,
(ii) des groupements carboxylates,
(iii) des groupements alcoxylés,
(iv) des groupements hydroxylés,
(v) des groupements acyloxyalkyles,
(vi) des groupements bisulfites.

Ces silicones sont connues en elles-mêmes ou sont entre autres décrites plus en détail dans le brevet français n° 2.653.016.

Les silicones organomodifiées peuvent également être choisies parmi les silicones organomodifiées par des groupements polyéthylène oxy et/ou polypropylèneoxy, alkylcarboxylique, 2-hydroxy-sulfonique, 2-hydroxyalkylthiosulfate et acylamidoalkyle.

Les compositions conformes à l'invention peuvent également contenir des isoparaffines ou poly-α-oléfines et des huiles perfluorées. On peut citer à cet effet :

. des isoparaffines de viscosité inférieure à 0,5 Pa.s, répondant à la formule :

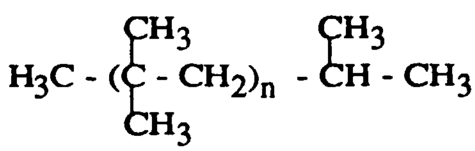

dans laquelle :

n est compris entre 2 et 16 et leurs mélanges avec des huiles de structure identique, dans laquelle n est supérieur à 18, et de préférence compris entre 18 et 40; de telles huiles sont vendues par la Société PRESPERSE INC. sous les dénominations "PERMETHYL 99A, 101A, 102A, 104A, 106A", ou par la Société ICI sous la dénomination "ARLAMOL HD". On peut citer aussi les "ISOPARS" vendus par la Société EXXON INC.

. des poly-α-oléfines de type polydécène hydrogénées ou non; de tels produits sont vendus par la Société ETHYL CORPORATION sous la dénomination "ETHYLFLO". On cite aussi les polyisobutylènes hydrogénés ou non.
. des huiles perfluorées telles que les perfluoropolyméthylisopropyléthers vendues sous la dénomination "FOMBLIN" par la Société MONTEFLUOS.

Les agents cosmétiques traitants sont présents dans les compositions conformes à l'invention, dans des proportions pouvant varier entre 0,01 et 10% en poids par rapport au poids total de la composition et de préférence entre 0,05 et 6% en poids.

Ces compositions, lorsqu'elles sont utilisées dans un traitement comportant un rinçage, peuvent également contenir des électrolytes tels que des sels de métaux alcalins, comme par exemple des sels de sodium, de potassium ou de lithium.

Ces sels sont choisis de préférence parmi les sulfates, les halogénures tels que le chlorure ou bromure ou les sels d'acides organiques, en particulier l'acétate ou lactate.

Sont également utilisés des sels de métaux alcalino-terreux, plus particulièrement les carbonate, silicate, nitrate, acétate, gluconate, pantohénate et lactate de calcium, magnésium ou strontium.

Ces électrolytes sont présents dans des concentrations variant entre 0,25 et 8% en poids par rapport au poids total de la composition.

Les compositions conformes à l'invention peuvent contenir d'autres produits actifs, tels que des agents anti-gras, des agents anti-séborrhéiques, des agents antipelliculaires, des agents pour lutter contre la chute des cheveux ou pour favoriser la repousse des cheveux, des produits antiacnéïques.

Les compositions peuvent également renfermer d'autres ingrédients habituellement utilisés en cosmétique, tels que des parfums, des colorants ayant pour fonction de colorer la composition elle-même, les cheveux ou la peau, des agents conservateurs, des agents séquestrants, des filtres solaires, des agents peptisants, ainsi qu'éventuellement des agents tensio-actifs anioniques, non-ioniques, amphotères ou leurs mélanges, dans des proportions inférieures à 10% et de préférence à 7%.

Il est entendu que lorsque les substances actives ou les ingrédients sont présents, ils ne doivent pas avoir un effet néfaste sur la production de la mousse obtenue grâce au terpolymère dérivé de vinyl lactame défini ci-dessus.

Les compositions conformes à l'invention sont conditionnées dans des dispositifs pressurisés en aérosol en présence des gaz propulseurs présents généralement dans des proportions ne dépassant pas 25% par rapport au poids total de la composition, et de préférence 15%.

On peut utiliser à titre de gaz propulseurs, le diméthyléther, l'air comprimé, le gaz carbonique, l'azote, l'oxyde nitreux; des hydrocarbures volatils tels que le butane, l'isobutane, le propane et leurs mélanges; les hydrocarbures halogénés, chlorés et/ou fluorés, non hydrolysables tels que les composés vendus sous les dénominations de "FREON" par la Société DU PONT DE NEMOURS, et plus particulièrement les fluorochlorohydrocarbures comme le dichlorodifluorométhane, Fréon 12, ou le dichlorotétrafluoroéthane ou Fréon 114. Ces agents propulseurs peuvent être utilisés seuls ou en mélange comme le mélange de Fréon 114/Fréon 12, dans des proportions comprises entre 40:60 et 80:20.

Les compositions introduites dans le dispositif aérosol, conformément à l'invention, peuvent se présenter sous forme de lotion, d'émulsion ou de dispersion qui, après distribution à partir du dispositif aérosol, forment des mousses à appliquer sur les cheveux ou la peau.

Les mousses distribuées à partir des dispositifs aérosol ayant la composition définie ci-dessus, peuvent être appliquées sous forme de composition après-shampooing, de produit à rincer à appliquer avant ou après coloration ou décoloration, avant ou après permanente ou défrisage, comme agent pour les mises en plis ou le brushing ou sous forme de produit dont l'application n'est pas suivie de rinçage tel que les mousses de coiffage.

Les mousses conformes à l'invention sont de préférence utilisées sans que leur application soit suivie d'un rinçage.

Ces compositions peuvent également être utilisées pour la restructuration des cheveux, les permanentes ou la coloration et décoloration des cheveux.

Leur pH est généralement compris entre 3 et 10 et ajusté à l'aide d'un agent alcalinisant ou acidifiant utilisé habituellement en cosmétique.

Lorsque les mousses conformes à l'invention sont destinées à être utilisées pour le coiffage, la mise en forme ou la mise en plis des cheveux, elles comprennent généralement en solution ou dispersion aqueuse ou hydroalcoolique contenant le terpolymère dérivé de vinyl lactame défini ci-dessus, un ou plusieurs polymères cosmétiques tels que définis ci-dessus.

Lorsque les mousses conformes à l'invention sont utilisées pour réaliser les permanentes, elles renferment outre le terpolymère de vinyl lactame défini ci-dessus et des polymères cosmétiques, soit un agent réducteur, soit un agent oxydant, suivant qu'elles constituent la première ou la seconde phase de la permanente.

Lorsque la mousse est appliquée sur la peau, elle contient différents adjuvants actifs tels que définis ci-dessus, suivant l'application envisagée.

Les exemples suivants sont destinés à illustrer l'invention.

EXEMPLE 1

On prépare la composition suivante :

| | |
|---|---|
| - Terpolymère vinylpyrrolidone/acide acrylique/méthacrylate de lauryle, vendu sous la dénomination "ANIONIC VP TERPOLYMER" par la Société ISP | 1 g |
| - Copolymère vinylméthyléther/anhydride maléique monoestérifié avec le butanol, vendu à 50% de matière active (MA) dans l'éthanol, sous la dénomination "GANTREZ ES 425" par la Société GAF | 1 g |
| - 2-amino-2-méthyl 1-propanol qs pH=6,5 | |
| - Polydiméthylsiloxane oxyéthyléné, à groupements amide, vendu sous la dénomination "SILWAX DCA-100" par la Société SILTECH | 0,5 g |
| - Eau déminéralisée | qsp 100 g |
| **Conditionnement aérosol** | |
| - Composition ci-dessus | 90 g |
| - Mélange ternaire : butane-isobutane > 55% -propane, vendu sous la dénomination "AEROGAZ 3,2 N" par la Société ELF-AQUITAINE | 10 g |
| | Total 100 g |

Cette composition est conditionnée dans un dispositif aérosol. Il se forme après expansion à l'air une mousse, onctueuse au toucher et stable.

Elle est appliquée sur les cheveux, et disparaît après massage. Les cheveux ainsi traités sont particulièrement doux au toucher et la coiffure a une bonne tenue.

EXEMPLE 2

On prépare la composition suivante :

| | |
|---|---|
| - Terpolymère vinylpyrrolidone/acide acrylique/méthacrylate de lauryle, vendu sous la dénomination "ANIONIC VP TERPOLYMER" par la Société ISP, neutralisé à 100% par le 2-amino 2-méthyl 1-propanol | 2 g avant neutralisation |
| - Copolymère polyvinylpyrrolidone quaternisé ayant un PM de 100.000, vendu à 50% de MA sous la dénomination "GAFQUAT 734" par la Société GAF | 0,5 g MA |
| - Hydrolysat de protéine de soja quaternisée, vendu en solution aqueuse à 30% sous la dénomination "CROQUAT SOYA" par la Société CRODA | 0,5 g MA |
| - Alcool éthylique | 8,5 g |
| - Parfum, conservateur qs | |
| - Eau déminéralisée | qsp 100 g |
| **Conditionnement aérosol** | |
| - Composition ci-dessus | 85 g |
| - Mélange ternaire : butane-isobutane > 55% -propane, vendu sous la dénomination "AEROGAZ 3,2 N" par la Société ELF-AQUITAINE | 15 g |
| | Total 100 g |

Cette composition est utilisée dans les mêmes conditions que celle de l'exemple 1. On obtient des résultats similaires sur les cheveux.

EXEMPLE 3

On prépare la composition suivante.

| | |
|---|---|
| - Terpolymère vinylpyrrolidone/acide acrylique/méthacrylate de lauryle, vendu sous la dénomination "ANIONIC VP TERPOLYMER" par la Société ISP, neutralisé à 100% par le 2-amino 2-méthyl 1-propanol | 0,5g avant neutralisation |
| - Copolymère d'hydroxyéthylcellulose greffé par voie radicalaire par du chlorure de diallyl diméthyl ammonium, vendu sous la dénomination "CELQUAT L200" par la Société NATIONAL STARCH | 0,5 g |
| - Chlorure de diméthylcétylhydroxyéthylammonium | 1 g |
| - Acide lactique qs pH=5 | |
| - Parfum, conservateur qs | |
| - Eau déminéralisée | qsp 100 g |
| **Conditionnement aérosol** | |
| - Composition ci-dessus | 90 g |
| - Mélange ternaire : butane-isobutane > 55% -propane, vendu sous la dénomination "AEROGAZ 3,2 N" par la Société ELF-AQUITAINE | 10 g |
| | Total 100 g |

La composition conditionnée dans un dispositif aérosol forme une mousse après expulsion à l'air.

Elle est appliquée sur des cheveux humides, lavés au préalable; après massage pour répartir la mousse dans la chevelure, et 5 minutes de pose, on rince à l'eau, on sèche et on met en forme la chevelure. Les cheveux séchés sont brillants, doux au toucher et ont une bonne tenue dans le temps. Cette mousse peut également être appliquée sans procéder à un rinçage. On constate de bonnes propriétés de douceur et de tenue.

EXEMPLE 4

On prépare la composition suivante :

| | | |
|---|---|---|
| - Terpolymère vinylpyrrolidone/acide acrylique/mèthacrylate de lauryle, vendu sous la dénomination "ANIONIC VP TERPOLYMER" par la Société ISP | | 1 g |
| - Copolymère vinylméthyléther/anhydride maléique monoestérifié avec le butanol, vendu à 50% de matière active (MA) dans l'éthanol sous la dénomination "GANTREZ ES 425" par la Société GAF | | 1 g |
| - 2-amino 2-méthyl 1-propanol qs pH=6,3 | | |
| - Polydiméthylsiloxane oxyéthyléné, vendu sous la dénomination "SILWET L7602" par la Société UNION CARBIDE | | 0,5 g |
| - Eau déminéralisée | qsp | 100 g |
| **Conditionnement aérosol** | | |
| - Composition ci-dessus | | 90 g |
| - Mélange ternaire : butane-isobutane > 55% -propane, vendu sous la dénomination "AEROGAZ 3,2 N" par la Société ELF-AQUITAINE | | 10 g |
| | Total | 100 g |

On utilise la mousse obtenue avec cette composition dans les mêmes conditions que dans l'exemple 1. Les résultats sont similaires.

<u>EXEMPLE 5</u>

On prépare la composition suivante :

| | | |
|---|---|---|
| - Terpolymère vinylpyrrolidone/acide acrylique/méthacrylate de lauryle, vendu sous la dénomination "ANIONIC VP TERPOLYMER" par la Société ISP, neutralisé à 100% par le 2-amino 2-méthyl 1-propanol | | 1,5 g avant neutralisation |
| - Nicotinate de méthyle | | 0,1 g |
| - Parfum, conservateur qs | | |
| - Eau déminéralisée | qsp | 100 g |
| **Conditionnement aérosol** | | |
| - Composition ci-dessus | | 90 g |
| - Mélange ternaire : butane-isobutane > 55% -propane, vendu sous la dénomination "AEROGAZ 3,2 N" par la Société ELF-AQUITAINE | | 10 g |
| | Total | 100 g |

La mousse obtenue après expansion à l'air à partir du dispositif aérosol est utilisée pour le traitement anti-chute des cheveux.
Elle est appliquée sur la chevelure et disparaît après massage. On constate que les cheveux traités sont doux au toucher et ont une bonne tenue après coiffage.
On constate après 2 mois de traitement une diminution de la chute des cheveux.

**Revendications**

1. Utilisation comme agent de moussage unique ou complémentaire dans des compositions aqueuses de traitement de la peau ou des cheveux, conditionnées dans un dispositif aérosol susceptibles de former après expansion à l'air une mousse, d'un terpolymère constitué de 25 à 90% de vinyl lactame, de 1 à 55% d'acide carboxylique insaturé

et de 1 à 20% d'acrylate ou de méthacrylate d'alkyle comportant au moins 6 atomes de carbone.

**2.** Utilisation selon la revendication 1, caractérisée par le fait que les acides carboxyliques insaturés sont choisis parmi l'acide acrylique, l'acide méthacrylique, l'acide itaconique ou l'acide crotonique.

**3.** Utilisation selon la revendication 1 ou 2, caractérisée par le fait que le vinyl lactame est la 2-vinylpyrrolidone.

**4.** Utilisation selon l'une quelconque des revendications 1 à 3, caractérisée par le fait que les esters acrylique ou méthacrylique comportent 8 à 18 atomes de carbone.

**5.** Utilisation selon la revendication 4, caractérisée par le fait que les radicaux alkyle des esters acrylique ou méthacrylique sont choisis parmi les radicaux 2-éthylhexyle, octyle, lauryle et stéaryle.

**6.** Composition destinée au traitement des cheveux ou de la peau, conditionnée dans un dispositif pressurisé en aérosol en présence d'un agent propulseur, caractérisée par le fait qu'elle contient dans un milieu aqueux cosmétiquement acceptable, au moins un terpolymère constitué de 25 à 90% de vinyl lactame, de 1 à 55% en poids d'acide carboxylique insaturé, et de 1 à 20% d'acrylate ou de méthacrylate d'alkyle comportant au moins 6 atomes de carbone de façon à former une mousse après expansion à l'air.

**7.** Composition selon la revendication 6, caractérisée par le fait que le terpolymère est tel que défini dans l'une quelconque des revendications 2 à 5.

**8.** Composition selon l'une quelconque des revendications 6 ou 7, caractérisée par le fait que le milieu cosmétique aqueux contient de l'eau ou un mélange d'eau et d'un solvant cosmétiquement acceptable présent dans une quantité telle à permettre l'obtention avec le terpolymère dérivé de vinyl lactame d'une mousse ayant une masse volumique inférieure ou égale à 0,3 g/cm$^3$ à 20°C et ayant une stabilité supérieure à 5 minutes.

**9.** Composition selon l'une quelconque des revendications 6 à 8, caractérisée par le fait qu'elle contient des agents traitants choisis parmi les agents tensio-actifs cationiques, les polymères cationiques, les polymères anioniques, les polymères non-ioniques et les polymères amphotères.

**10.** Composition selon l'une quelconque des revendications 5 à 9, caractérisée par le fait qu'elle contient des silicones volatiles ou non volatiles choisies parmi les polyalkylsiloxanes, les polyarylsiloxanes, les polyalkylarylsiloxanes, des silicones organomodifiées par des groupements polyéthylèneoxy et/ou polypropylèneoxy, thiol, carboxylate, alcoxy, hydroxyle, acyloxyalkyle, alkylcarboxylique, 2-hydroxysulfonique, 2-hydroxyalkylthiosulfate et acylamidoalkyle.

**11.** Composition selon l'une quelconque des revendications 5 à 10, caractérisée par le fait qu'elle contient en outre une isoparaffine, une poly-$\alpha$-oléfine ou une huile perfluorée.

**12.** Composition selon l'une quelconque des revendications 6 à 11, caractérisée par le fait que le polymère dérivé de vinyl lactame est présent dans des proportions comprises entre 0,05 et 10% en poids et de préférence entre 0,2 et 3% en poids par rapport au poids total de la composition.

**13.** Composition selon l'une quelconque des revendications 6 à 12, caractérisée par le fait que la composition contient un agent traitant présent dans des proportions comprises entre 0,01 et 10% en poids par rapport au poids total de la composition.

**14.** Composition selon l'une quelconque des revendications 6 à 13, caractérisée par le fait qu'elle contient des électrolytes choisis parmi les sulfates, les halogénures ou les sels d'acides organiques de métaux alcalins ou alcalino-terreux.

**15.** Composition selon l'une quelconque des revendications 6 à 14, caractérisée par le fait qu'elle contient des agents actifs cosmétiques ou dermatologiques.

**16.** Composition selon l'une quelconque des revendications 6 à 15, caractérisée par le fait que les agents actifs sont choisis parmi les agents anti-gras, les agents antiséborrhéiques, les agents antipelliculaires, les agents antiacnéiques, des produits pour lutter contre la chute des cheveux ou pour favoriser la repousse des cheveux.

17. Composition selon l'une quelconque des revendications 6 à 16, caractérisée par le fait qu'elle contient également des parfums, des colorants ayant pour fonction de colorer la composition elle-même ou les cheveux ou la peau, des agents conservateurs, des agents séquestrants, des agents adoucissants, des filtres solaires, des agents peptisants.

18. Composition selon l'une quelconque des revendications 6 à 17, caractérisée par le fait qu'elle contient également des agents tensioactifs anioniques, non-ioniques, amphotères ou leurs mélanges, dans des proportions ne dépassant pas 10%.

19. Composition selon l'une quelconque des revendications 6 à 18, caractérisée par le fait qu'elle est appliquée sous forme de composition après-shampooing, de mousse à rincer à appliquer avant ou après coloration ou décoloration, avant ou après permanente ou défrisage, de mousse de mise en plis, de brushing, de mousse non rincée après application, de composition restructurante, de permanente, de coloration ou de décoloration.

20. Procédé de traitement cosmétique des cheveux ou de la peau, caractérisé par le fait que l'on applique sur la peau ou les cheveux, au moins une mousse résultant de l'expansion à l'air de la composition telle que définie dans l'une quelconque des revendications 6 à 19, cette application étant éventuellement suivie d'un rinçage.

21. Composition destinée au traitement thérapeutique de la peau, caractérisée par le fait qu'elle contient dans une composition telle que définie dans l'une quelconque des revendications 6 à 18 et formant après expansion à l'air une mousse, au moins une substance active au niveau dermatologique.

**Claims**

1. Use as sole or additional foaming agent in aqueous compositions for treating the skin or the hair, which are packaged in an aerosol device and are capable of forming a foam after expansion into the air, of a terpolymer consisting of 25 to 90% of vinyl lactam, of 1 to 55% of unsaturated carboxylic acid and of 1 to 20% of alkyl acrylate or methacrylate containing at least 6 carbon atoms.

2. Use according to Claim 1, characterized in that the unsaturated carboxylic acids are chosen from acrylic acid, methacrylic acid, itaconic acid or crotonic acid.

3. Use according to Claim 1 or 2, characterized in that the vinyl lactam is 2-vinylpyrrolidone.

4. Use according to any one of Claims 1 to 3, characterized in that the acrylic or methacrylic esters contain 8 to 18 carbon atoms.

5. Use according to Claim 4, characterized in that the alkyl radicals of the acrylic or methacrylic esters are chosen from 2-ethylhexyl, octyl, lauryl and stearyl radicals.

6. Composition intended for treating the hair or the skin, which is packaged in a pressurized device as an aerosol in the presence of a propellent, characterized in that it contains, in a cosmetically acceptable aqueous medium, at least one terpolymer consisting of 25 to 90% of vinyl lactam, of 1 to 55% by weight of unsaturated carboxylic acid and of 1 to 20% of alkyl acrylate or methacrylate containing at least 6 carbon atoms, so as to form a foam after expansion into the air.

7. Composition according to Claim 6, characterized in that the terpolymer is as defined in any one of Claims 2 to 5.

8. Composition according to either of Claims 6 and 7, characterized in that the aqueous cosmetic medium contains water or a mixture of water and a cosmetically acceptable solvent which is present in such an amount as to make it possible to obtain with the vinyl lactam-derived terpolymer a foam having a density of less than or equal to 0.3 $g/cm^2$ at 20°C and having a stability of greater than 5 minutes.

9. Composition according to any one of Claims 6 to 8, characterized in that it contains treating agents chosen from cationic surface-active agents, cationic polymers, anionic polymers, nonionic polymers and amphoteric polymers.

10. Composition according to any one of Claims 5 to 9, characterized in that it contains volatile or non-volatile silicones chosen from polyalkylsiloxanes, polyarylsiloxanes, polyalkylarylsiloxanes, silicones which are organically modified

with polyethylenoxy and/or polypropylenoxy, thiol, carboxylate, alkoxy, hydroxyl, acyloxyalkyl, alkylcarboxylic, 2-hydroxysulphonic, 2-hydroxyalkylthiosulphate and acylamidoalkyl groups.

**11.** Composition according to any one of Claims 5 to 10, characterized in that it additionally contains an isoparaffin, a poly-$\alpha$-olefin or a perfluoro oil.

**12.** Composition according to any one of Claims 6 to 11, characterized in that the vinyl lactam-derived polymer is present in proportions of between 0.05 and 10% by weight and preferably of between 0.2 and 3% by weight relative to the total weight of the composition.

**13.** Composition according to any one of Claims 6 to 12, characterized in that the composition contains a treating agent which is present in proportions of between 0.01 and 10% by weight relative to the total weight of the composition.

**14.** Composition according to any one of Claims 6 to 13, characterized in that it contains electrolytes chosen from sulphates, halides or organic acid salts of alkali metals or of alkaline-earth metals.

**15.** Composition according to any one of Claims 6 to 14, characterized in that it contains cosmetic or dermatological active agents.

**16.** Composition according to any one of Claims 6 to 15, characterized in that the active agents are chosen from anti-greasy agents, anti-seborrheic agents, anti-dandruff agents, anti-acne agents, products for combating hair loss or for promoting the regrowth of the hair.

**17.** Composition according to any one of Claims 6 to 16, characterized in that it also contains fragrances, dyes for the purpose of colouring the composition itself or the hair or the skin, preserving agents, sequestering agents, emollients, sunscreen agents and peptizing agents.

**18.** Composition according to any one of Claims 6 to 17, characterized in that it also contains anionic, nonionic or amphoteric surface-active agents or mixtures thereof, in proportions not exceeding 10%.

**19.** Composition according to any one of Claims 6 to 18, characterized in that it is applied in the form of a conditioner composition, a foam to be rinsed which is to be applied before or after dyeing or bleaching, before or after permanent waving or hair straightening, a hair setting foam, a blow-drying foam, a foam which is not rinsed out after application, a restructuring composition, a permanent-waving composition, a dyeing composition or a bleaching composition.

**20.** Process for the cosmetic treatment of the hair or of the skin, characterized in that at least one foam resulting from the expansion into the air of the composition as defined in any one of Claims 6 to 19 is applied to the skin or to the hair, this application optionally being followed by a rinsing operation.

**21.** Composition intended for the therapeutic treatment of the skin, characterized in that it contains, in a composition as defined in any one of Claims 6 to 18 and which forms a foam after expansion into the air, at least one dermatologically active substance.

## Patentansprüche

**1.** Verwendung eines Terpolymers, das aus 25 bis 90 % Vinyllactam, 1 bis 55 % einer ungesättigten Carbonsäure und 1 bis 20 % Alkylacrylat oder Alkylmethacrylat mit mindestens 6 Kohlenstoffatomen besteht, als einzigen oder zusätzlichen Schaumbildner in wäßrigen Zusammensetzungen zur Behandlung der Haut oder der Haare, die in einer Aerosolvorrichtung konfektioniert sind und nach Expansion an Luft einen Schaum bilden können.

**2.** Verwendung nach Anspruch 1, dadurch gekennzeichnet, daß die ungesättigten Carbonsäuren unter Acrylsäure, Methacrylsäure, Itaconsäure oder Crotonsäure ausgewählt sind.

**3.** Verwendung nach Anspruch 1 oder 2, dadurch gekennzeichnet, daß das Vinyllactam das 2-Vinylpyrrolidon ist.

**4.** Verwendung nach einem der Ansprüche 1 bis 3, dadurch gekennzeichnet, daß die Acrylester oder Methacrylester 8 bis 18 Kohlenstoffatome enthalten.

5. Verwendung nach Anspruch 4, dadurch gekennzeichnet, daß die Alkylgruppen der Acryl- oder Methacrylester unter 2-Ethylhexyl, Octyl, Lauryl und Stearyl ausgewählt sind.

6. Zusammensetzung zur Behandlung der Haare oder der Haut, die in einer Vorrichtung konfektioniert ist, welche als Aerosol in Gegenwart eines Treibmittels unter Druck gesetzt ist, dadurch gekennzeichnet, daß sie mindestens ein Terpolymer enthält, das aus 25 bis 90 % Vinyllactam, 1 bis 55 % einer ungesättigten Carbonsäure und 1 bis 20 % Alkylacrylat oder Alkylmethacrylat mit mindestens 6 Kohlenstoffatomen besteht, in einem kosmetisch akzeptablen, wäßrigen Medium enthält, so daß nach Expansion an Luft ein Schaum gebildet wird.

7. Zusammensetzung nach Anspruch 6, dadurch gekennzeichnet, daß das Terpolymer ein Terpolymer nach einem der Ansprüche 2 bis 5 ist.

8. Zusammensetzung nach einem der Ansprüche 6 oder 7, dadurch gekennzeichnet, daß das wäßrige kosmetische Medium Wasser oder ein Gemisch aus Wasser und einem kosmetisch akzeptablen Lösungsmittel enthält, das in einer solchen Menge vorliegt, daß mit dem von Vinyllactam abgeleiteten Terpolymer ein Schaum mit einer Dichte von höchstens 0,3 $g/cm^3$ bei 20 °C und einer Stabilität über 5 min hergestellt werden kann.

9. Zusammensetzung nach einem der Ansprüche 6 bis 8, dadurch gekennzeichnet, daß sie Behandlungsmittel enthält, die unter den kationischen grenzflächenaktiven Stoffen, kationischen Polymeren, anionischen Polymeren, nichtionischen Polymeren und amphoteren Polymeren ausgewählt sind.

10. Zusammensetzung nach einem der Ansprüche 5 bis 9, dadurch gekennzeichnet, daß sie flüchtige oder nicht flüchtige Silicone enthält, die unter den Polyalkylsiloxanen, Polyarylsiloxanen, Polyalkylarylsiloxanen, mit Polyethylenoxygruppen und/oder Polypropylenoxygruppen, Thiol, Carboxylat, Alkoxy, Hydroxy, Acyloxyalkyl, Alkylcarboxy, 2-Hydroxysulfonsäure, 2-Hydroxyalkylthiosulfat und Acylamidoalkyl organomodifizierten Siliconen ausgewählt sind.

11. Zusammensetzung nach einem der Ansprüche 5 bis 10, dadurch gekennzeichnet, daß sie ferner ein Isoparaffin, ein Poly-$\alpha$-olefin oder ein perfluoriertes Öl enthält.

12. Zusammensetzung nach einem der Ansprüche 6 bis 11, dadurch gekennzeichnet, daß das von Vinyllactam abgeleitete Polymer in Mengenanteilen im Bereich von 0,05 bis 10 Gew.-% und vorzugsweise im Bereich von 0,2 bis 3 Gew.-%, bezogen auf das Gesamtgewicht der Zusammensetzung, vorliegt.

13. Zusammensetzung nach einem der Ansprüche 6 bis 12, dadurch gekennzeichnet, daß die Zusammensetzung ein Behandlungsmittel enthält, das in Mengenanteilen im Bereich von 0,01 bis 10 Gew.-%, bezogen auf das Gesamtgewicht der Zusammensetzung, vorliegt.

14. Zusammensetzung nach einem der Ansprüche 6 bis 13, dadurch gekennzeichnet, daß sie Elektrolyte enthält, die unter den Sulfaten, Halogeniden oder Salzen von organischen Säuren von Alkalimetallen oder Erdalkalimetallen ausgewählt sind.

15. Zusammensetzung nach einem der Ansprüche 6 bis 14, dadurch gekennzeichnet, daß sie kosmetisch oder dermatologisch wirksame Mittel enthält.

16. Zusammensetzung nach einem der Ansprüche 6 bis 15, dadurch gekennzeichnet, daß die Wirkstoffe unter den Antifettmitteln, Mitteln gegen Seborrhoe, Mitteln gegen Schuppen, Mitteln gegen Akne und Mitteln zur Bekämpfung von Haarausfall oder Mitteln zur Begünstigung des Haarwachstums ausgewählt sind.

17. Zusammensetzung nach einem der Ansprüche 6 bis 16, dadurch gekennzeichnet, daß sie ferner Parfums, Färbemittel, die die Zusammensetzung selbst oder die Haare oder die Haut färben sollen, Konservierungsmittel, Maskierungsmittel, reizlindernde Mittel, Sonnenschutzfilter und Peptisierungsmittel enthält.

18. Zusammensetzung nach einem der Ansprüche 6 bis 17, dadurch gekennzeichnet, daß sie ferner anionische, nichtionische oder amphotere grenzflächenaktive Stoffe oder deren Gemische in Mengenanteilen nicht über 10 % enthält.

19. Zusammensetzung nach einem der Ansprüche 6 bis 18, dadurch gekennzeichnet, daß sie in Form einer Zusammensetzung zum Auftragen nach der Haarwäsche, als Schaum, der ausgespült wird, vor oder nach einer

Färbung oder Entfärbung, vor oder nach einer permanenten Verformung oder Entkräuselung aufgetragen wird, Schaum für Wasserwellen oder Fönwellen, Schaum, der nach dem Auftragen nicht ausgespült wird, restrukturierende Zusammensetzung, Zusammensetzung für Dauerwellen, Zusammensetzung zum Färben oder Entfärben aufgetragen wird.

**20.** Verfahren zur kosmetischen Behandlung der Haare oder der Haut, dadurch gekennzeichnet, daß auf die Haut oder die Haare mindestens ein Schaum aufgebracht wird, der nach Expansion der Zusammensetzung nach einem der Ansprüche 6 bis 19 an Luft resultiert, wobei nach dem Auftragen ggf. gespült wird.

**21.** Zusammensetzung zur therapeutischen Behandlung der Haut, dadurch gekennzeichnet, daß sie in einer Zusammensetzung nach einem der Ansprüche 6 bis 18, die nach Expansion an Luft einen Schaum bildet, mindestens eine dermatologisch wirksame Substanz enthält.